(19) Europäisches Patentamt
European Patent Office
Office européen des brevets

(11) Publication number: 0 077 577 B1

(12) EUROPEAN PATENT SPECIFICATION

(45) Date of publication of patent specification: 04.12.85

(51) Int. Cl.[4]: G 03 C 1/68

(21) Application number: 82110677.0

(22) Date of filing: 09.06.80

(60) Publication number of the earlier application in accordance with Art. 76 EPC: 0 022 618

# (54) Photographic articles comprising a compound having acetylenic bonds and methods for producing the articles.

(30) Priority: 25.06.79 US 52007
21.01.80 US 113552

(43) Date of publication of application:
27.04.83 Bulletin 83/17

(45) Publication of the grant of the patent:
04.12.85 Bulletin 85/49

(84) Designated Contracting States:
BE CH DE FR GB IT LI NL SE

(56) References cited:
DE-A-1 940 691
FR-A-1 525 738
FR-A-2 214 703
FR-A-2 401 443

RESEARCH DISCLOSURE, no. 109, May 1973, pages 73-74, no. 10941, Havant, Hampshire (GB); "Dispersion of polyacetylenic compound in hydrophilic binder"

The file contains technical information submitted after the application was filed and not included in this specification

(73) Proprietor: UNIVERSITY PATENTS, INC.
537 Newtown Avenue P.O. Box 6080
Norwalk, CT 06852 (US)

(72) Inventor: Garito, Anthony Frank
106 Rock Rose Lane
Radnor Pennsylvania 19087 (US)

(74) Representative: Evans, David Charles et al
F.J. CLEVELAND & COMPANY 40-43, Chancery Lane
London, WC2A 1JQ (GB)

(56) References cited:
RESEARCH DISCLOSURE, no. 109, May 1973, pages 60-61, no. 10930, Havant, Hampshire (GB); "Fluorescing images produced by polyacetylenic materials"
MAKROMOL. CHEM., vol. 179, 1978, pages 1639-1642, Basel (CH); B TIEKE et al.: "The quantum yield of the topochemical photopolymerization of diacetylenes in multilayers"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 077 577 B1

Courier Press, Leamington Spa, England.

**IBM TECHNICAL DISCLOSURE BULLETIN, vol. 13, no. 6, November 1970, page 1717, New York (USA); R.A. COUTURE et al.: "Resist adhesion to hydrophilic surfaces"**
**IBM TECHNICAL DISCLOSURE BULLETIN, vol. 20, no. 4, September 1977, page 1445, New York (USA); G.J. COLLINI: "Wetting agent for resist application"**

**Description**

This invention relates to photographic and photolithographic articles and for methods of producing such articles. The invention is particularly concerned with articles comprising a substrate and a layer carried thereby of a photopolymerizable composition comprising a photopolymerizable di or tri-acetylenic monomer. The photopolymerizable nature of diacetylenes in multilayers is known, see for example, Journal of Polymer Science, Polymer Chemistry Edition Vol. 17, 1631—1644 (1979). This article discloses a series of amphiphilic diacetylene monocarbonic acids were synthesized and their ability to form monolayers at the air-water interphase was investigated. Acids with total number of carbon atoms greater than 20 and a melting point greater than 45°C form surface states suitable to be used for build-up of multilayers by the Langmuir-Blodgett technique. Using the LB technique, multilayers of defined thickness were built up on quartz substrates. The multilayers were polymerized by exposure to an ultra violet light source according to the mechanism of solid state polymerization of diacetylenes without destruction of the layer structure and with retention of the packing in the individual layers. Thus well-defined polymer multilayers were obtained. The monomer and polymer multilayers were characterised by ultra violet spectra, x-ray small-angle diffraction and interference microscopic and electron microscopic techniques.

These diacetylenic compositions offer vastly improved properties for photography and photolithography as compared with the materials known hitherto.

Photo-lithography finds its roots in 1852 when British Patent No. 565 was issued to Talbot for a bichromate sensitized gelatin coating which was useful for differential etching of copper ferric chloride. Since this time, numerous compositions and processes have been developed which serve to transfer a pattern of light or other radiant energy onto a substrate through the intermediation of a coating sensitive to such radiant energy. The art of photolithography has developed broadly in recent decades to include various modes of printing, etching, and image reproduction from macro to micro scales, and has fostered the development of entire new industries. See generally, Photo-Resist Materials and Processes by William DeForest, McGraw-Hill 1975.

One industry which has placed heavy reliance upon the use of photolithography is that of microstructure fabrication. This industry which produces structures imposed upon surfaces of thin films having extremely small dimensions is the basis for the production of information processing, electronics, switching, and other devices which rely upon "integrated" electronics, the fabrication and interconnection of large numbers of very small device structures on a single piece of silicon or other substrate.

Exemplary uses of photolithography employing what is commonly denominated in the art was photoresists, are described in Science, Volume 196, No. 4293, pp945—949, 27 May 1977, by R. W. Keyes. As taught in the Keyes article, a substrate, usually silicon, is caused to be coated with a layer of electrically non-conductive material, frequently silicon dioxide, usually by "growth" from the substrate itself through a suitable oxidizing process. The substrate is then further coated with one or more layers of photo-resist. At this time, selected portions of the photo-resist are exposed to a suitable form of radiant energy, which irradiation causes an alteration of the structure or physical characteristics of the portions of the resist thus exposed. Thus, electromagnetic energy such as light, especially ultraviolet light; X-radiation, lader radiation, etc. are particle radiation such as electron beam, particle beam, and plasma radiation may be utilized to alter the structure or physical characteristics of the portion of the photo-resist irradiated.

Following the irradiation of the selected portions of the photo-resist and concomitant alteration of the structure or physical characteristics thereof, a distinction may be drawn between the selected portions of the resist and those not selected based upon the altered structure or physical characteristics. Thus, the selected portions may be viewed as being either more or less suited to removal from the underlying substrate than are the unselected portions through any appropriate means. The selected portions may be either more or less soluble in a liquid medium, may have a higher or lower vapour pressure, may exhibit greater or lesser resistance to chemical attack, or may evidence any other differing structure or characteristic which may facilitate differential removal from the substrate as compared to the unselected portions. Based upon one or more of these distinctions, the selected (irradiated) portions of the photo-resist are either removed from the substrate while the unselected (unexposed) portions remain, or the selected portions are retained while the unselected portions are removed. By analogy with traditional photographic processes, the former process is carried out with photo-resists denominated as "positive" resists while the latter employ "negative" photo-resists.

The selection of certain portions of a photoresist for irradiation may be accomplished in various ways, all of which are well known to those skilled in the art. Masking is a common procedure which is performed by placing over the surface of a photo-resist-coated substrate (or composite substrate) a mask, usually formed photographically, and by causing the passage of radiation through the mask onto the resist layer. The mask is designed so as to have transmissive and non-transmissive areas corresponding to the portions of the resist layer which have been chosen for irradiation and for non-irradiation respectively. As is well known to those skilled in the art, the radiant energy used in conjunction

with the masking technique may benefit from being collimated, focused, monochromatized, or rendered coherent. Such persons will also recognize that all portions of the electromagnetic spectrum may be employed with the masking technique as may various forms of particle irradiation such as electron beams, ion beams, and plasma irradiation.

It is also well known to irradiate selected portions of a resist layer without the use of masking. Thus direct projection of a radiant energy beam, especially a laser, electron, or particle beam may be undertaken to irradiate the selected portions of the layer. Such projection is usually controlled by automated means and often by a computer. It is to be understood that the means and mechanisms for irradiating selected portions of the photoresist layer are well known in the art. See, for example, proceedings of the IEEE, volume 62, No. 10, pp 1361—1387, October, 1974, Henry I. Smith, Fabrication Techniques for Surface-Acoustic-Wave and Thin-Film Optical Devices; X-Ray Optics: Applications to Solids (Ed. H. J. Queisser); E. Spiller and R. Feder, X-ray Lithography, pp 35—92 Springer (1977); and Ann. Rev. Mat. Sci., vol. 6, pp 267—301, L. F. Thompson and R. E. Kerwin, Polymer Resist Systems for Photo- and Electron Lithography.

It may be seen that following the irradiation of the selected portions of the photo-resist and the selected removal either of the selected portions or of those portions not selected, a pattern will remain on the substrate formed of irradiated or non-irradiated photo-resist depending upon whether a negative or positive resist formulation was employed. This photo-resist pattern will alternate with exposed substrate, usually silicon oxide. The exposed silicon oxide (or other) substrate layer segments or portions are then subject to treatment. In nearly all cases, the oxide layer is removed or etched away in manners well known to those skilled in the art. The etching away of the silicon oxide layer is designed to expose the underlying silicon substrate to treatment and processing. The etching of the silicon oxide layer is intended to be accomplished only in the areas not covered with portions of photo-resist. The photo-resist must therefore be relatively insensitive to the etching means employed to remove the exposed oxide portions.

After the etching off of the oxide layer the remaining photo-resist is removed to yield a pattern of exposed silicon and silicon oxide or other substrate formulation. The exposed silicon is then treated to alter its electrical characteristics, frequently through processes denominated as "doping". "Doping", which is well known to those skilled in the art, causes elements such as phosphorous or boron to be diffused into the exposed silicon or other substrate material. The treatment of the article after the removal of the photo-resist is not directly related to this embodiment of the invention and is well known to those skilled in the art. The treated article may subsequently be re-coated with photoresistive materials, exposed selectively, and caused to undergo differential removal of resist, etching, and treatment one or more additional times to result in complex surface structures on the substrate. A selectively exposed and removed resist layer may also be used for the deposition of metal or other conductive materials onto the surface of the substrate to serve as electrical contacts with the variously treated surface areas as is well known to those skilled in the art.

It is to be understood that the foregoing background is not intended as a rigorous delination of the metes and bounds of the microfabrication art and that numerous variations, both of materials and processes are known. It should be further understood that the term "photo-resist" has been adopted by those skilled in the art as a generic term descriptive of all forms of radiation-sensitive coatings useful in lithography. Thus, the term embraces coatings useful for microfabrication, etching, printing, and compositing as well as numerous others. Similarly, the term "photo-resist" has been adopted for those coatings which are sensitive to particulate radiation such as electron beams.

It is apparent from the foregoing that the characteristics of the photo-resists useful in a particular photolithographic process is of central importance to the success of that process.

Further, in DE—A—1946690 and Research Disclosure No: 109, May 1973 P 73—74 and Page 60—61 various crystalline diacetylenes are disclosed in layers as assubstrate for photographic purposes.

It will be appreciated that the resolution of a resultant photographic article is a function of the size of the microcrystalline domains.

It will be appreciated, therefore, that there is a need for a photo-resist which is capable of ultra-fine resolution, exhibits a high response to incident radiation, easily applied to substrates, coats such substrates and adheres to them firmly until removal is desired. Furthermore, it is desirable that the resist, when exposed to incident radiation, should undergo a substantial change in physical properties, thus to provide a clear distinction between the exposed and unexposed portions so as to facilitate the selective removal of one or the other from the substrate when desired.

According to the present invention, there is provided a photographic or photolithographic article comprising a substrate, and at least one monomolecular layer carried by said substrate of a photopolymerizable composition, said layer comprising a material having at least two conjugated acetylenic bonds, characterised in that said layer is formed of a plurality of contiguous discrete domains.

The invention also includes a first method of making the article of the invention, which method comprises laying down a suitable substrate, forming at least one monomolecular layer of a photopolymerizable composition thereon, said composition comprising a material having at least

two conjugated actylenic bonds, characterized in that said article is heated to a temperature just below the melting point of said material to establish discrete domains in the layer after coating.

The invention also provides a second method of making the article of the invention which method comprises laying down a suitable substrate, forming at least one monomolecular layer of a photopolymerizable composition thereon, said composition comprising a material having at least two conjugated actylenic bonds, characterized in that the forming of a molecular layer is carried out using the Langmuir-Blodgett film forming technique, and in that the pH, the temperature and/or the specific gravity of the water sub-phase thereof is adjusted to cause or allow formation of domains in the resulting layer.

In one embodiment of the present invention, the article may comprise a plurality of domains within which a majority of the molecules of the monomeric material are oriented in a regular array. The orientation of the molecular array in one of said domains is preferably transverse to the orientation of the molecular arrays of the majority of domains contiguous thereto. It is preferred that the domains have an average dimension in the plane of the layer of not more than 1,000 nm and more preferably, not more than 300 nm. In one embodiment of the invention the domains may have an average dimension of less than 100 nm.

The discrete domains may include molecules of lower polymerisability than said material. The said material may further include molecules which have a steric structure or substituent groups which reduce the polymerisability thereof. The photopolymerizable layer may also include group which bond covalently with the substrate; these groups may be silyl or siloxyl groups. In a further embodiment of the invention, a bonding layer may be interposed between the layer of a photopolymerizable composition and the substrate therefor, said bonding layer being covalently bonded at least to the layer of a photopolymerisable composition. The bonding layer may comprise a compound having the general formula

XI. $(R_{11}—O)_3—Si(R_{12})—Z$

where the $R_{11}$ groups may be the same or different and are hydrocarbyl groups having from 1 to about 6 carbon atoms, where $R_{12}$ is a hydrocarbyl group having from 1 to about 6 carbon atoms, and Z is any substituent which may be covalently bonded with the material having at least two conjugated acetylene bonds.

A typical example of such a compound is 3 aminopropyletriethoxysilane. The layer may further include a nitrosodimer to retard thermally induced polymerisation without interferring with the domain structure or with the photopolymerisability of the material per se.

As indicated above, in order to advance the art of technology know as "the silicon chip" technology as discussed above, it is clearly apparent that photoresist articles are necessary which have greatly improved efficiency, permeability, structural regularity, resolution, contrast and adhesion to substrates.

It will be appreciated that an improvement in resolution means that smaller structures can be prepared on a given size of micro chips surface. The particular problem hitherto has been that with an amorphous polymer resist, they undergo a physical "relaxation" phenomena which leads to microstructural line indistinction and to a lowering of resolution. When an image is projected on to such a photoresistive layer, the image of the microstructural line pattern will be sharp and extremely distinct. The exposed areas of the photopolymerisable composition will polymerise and polymerisation will occur across the boundaries of the microstructural line image projected onto the surface, thus resulting in a blurring of the image on removal of the coating during processing or "development" of the photoresistive image.

The present invention seeks to overcome this particular problem by seeking to form discrete "domains" of groups of photopolymerisable molecules within the layer into specific "domains". As hereinafter described in order for the photopolymerisation reaction to take place effectively, the molecules need to be oriented at least to some extent for that photopolymerisation reaction to proceed.

The Applicants have realised that by confining and controlling the areas of orientation, the resolution of the resulting photoresist can be controlled. Thus, by reducing the size of the "domains" to the order of 100 nm or less, the Applicants have found that they are able to improve very significantly the micro-structural resolution of photoresists images over and above that described hitherto. This permits the use of shorter wavelengths radiation which in itself permits improvements in the resolution of the microstructural lines in the photoresist image. For example, X radiation in use at the present time on prior art photoresistive compositions permits resolution of structures of not much below 1000 nm. In contradistinction, the present invention permits the resolution of microstructures line of the order of below 1,000 nm usually below 200 to 300 nm in extreme circumstances, the resolution of structures as small as 100 nm may be obtained. This dramatic improvement in resolution leads, therefore, to an immediate increase in the number of microstructures which may be fabricated on a given surface area of substrate and this in term permits the reduction in size of articles such as microchips made thereby and leads to a substantial economic advantage in all forms of electronic control equipment relient upon such articles.

Following is a description by way of example only and with reference to the accompanying

drawings of methods of carrying the invention into effect.

In the drawings:—

Fig. 1 is a schematic representation of the polymerization of the compositions of the invention according to the processes of the invention, whereby a mechanism is postulated. The regularity of assemblages of monomer and of the resulting polymer is shown.

Fig. 2 is a representational view of certain embodiments of the present invention, especially photographic film. Thus 1 represents a coating comprising the materials of the invention, while 2 refers to a substrate which may comprise either one or a plurality of layers. It is to be understood that coating 1 may be monolayer, multilayer, or other coating. Additionally, substrate 2 may comprise inhomogeneous material or aggregates. In one embodiment, substrate 2 comprises a flexible film while coating 1 is one or more layers, of diacetylenic materials; in another, 2 is doped silica, and

Fig. 3 is intended to suggest the domain microstructure of certain preferred embodiments of the invention. Thus, domains such as those indicated by 3, are present, which domains have substantially regular arrays of constituent molecules. The orientations of these arrays are to be implied from the hatching placed thereupon. Reference No. 4 is intended to suggest the average dimension of a domain according to preferred forms of the invention.

The invention provides for novel means of photography, macro and micro lithography, printing, etching and engraving and for the elaboration and fabrication of micro structures, especially those useful in the electronics and integrated electronics fields.

The articles of the invention provide for superior performance in the elaboration and fabrication of electronic microstructures, as requiring lesser amounts of radiant energy in use, and as enabling a significantly more detailed resistive pattern structure to be composed upon the surface of a substrate. Thus, the processes utilize a genus of photo-resistive material which is energy efficient. This energy efficiency may be demonstrated by comparison of an X-ray photoresist recording to the present invention with typical members currently in use in the art. It has been reported that from 1 to about 60 joules per cubic centimeter absorbed dose is required for currently used negative X-ray resists. In contrast, the articles of the invention require only about 0.001—0.01 joules per cubic centimeter for polymerization. This high efficiency is manifested by short exposure times and concomitant savings in radiant energy output, in time, and in production costs.

The layers used in the invention are negative in mechanism, that is, upon exposure to radiant energy, the polymerize to form areas of polymer which are differentially retained upon exposure to a solvent as compared to the unexposed areas of photo-resist. The layers have a physical and electronic structure which is uniquely suited to polymerization thus exhibiting an excellent overall efficiency or quantum yield of polymerization upon exposure to radiant energy.

The regularity exhibited by the compositions is such that extremely fine exposure patterns may be resolved in the photo-resistive layers formed thereby. For example, X-ray photoresistive layers currently in use permit resolution of structures on the order of 1,000 nm and to 200—300 nm in extreme cases. The articles of this invention permit resolution of structure as small as 10 nm. This improvement in resolution leads to an immediate increase in the number of microstructures which may be fabricated on a given surface area of substrate. This increase permits the reduction in size of articles made thereby and leads to substantially economic advantage.

As stated above, amorphous polymer resists undergo physical "relaxation" phenomena in certain systems which lead to microstructural line indistinction and blurring. Additionally, it is known that most systems known to the prior art experience some dissolution of polymer intended to remain during the dissolution and removal step thus leading to edge undercutting. The articles of the present invention are ideally suited to overcome these problems however. Upon exposure to incident radiation the compositions polymerize into regular polymers with great facility and with high efficiency. The resulting polymers possess physical properties which are vastly different from the unpolymerized compositions. Especially significant is the large difference in solubility between the species; indeed the polymerized compositions are virtually insoluble in commonly employed solvents as compared with the unreacted compositions. As a result, therefore, the unexposed, and consequently, unpolymerized, areas are removed almost entirely by exposure to solvent while the exposed regions remain almost totally intact and in place. At the same time, the extreme regularity of the polymerization process together with the domain structure of the photopolymerisable layer and the geometric similarity of the polymers thus formed to their constituent monomers minimizes the "relaxation" experienced by the system upon polymerization. Thus line definition is sharp and blurring avoided.

The compositions need not employ a sensitizer although one may be employed if desired. Furthermore, the methods of the invention may employ coating techniques which lead to extreme regularity of substrate coverage and result in similar regularity of lithographic resolution and clarity.

One article includes compounds which liberate radical trapping agents upon the application of heat. Such compounds, such as dimers of nitroso compounds which are known in the ethylenic system (see U.S. Patent 4,168,982) serve to reduce thermal polymerization and avoid "undercutting". Diacetylenic systems are particularly benefitted by their inclusion.

The compositions employed in the methods of

the invention demonstrate superior adhesion to substrates and exhibit a high degree of dissimilarity in physical properties upon polymerization thus leading to ease of processing and superior results. Indeed a preferred article of the invention employs siloxyl moieties to form covalent bonds between the photoresistive layer and the substrate to result in extremely good adhesion between the substrate and the resist. Other forms of covalent bonding may also be employed.

The utilization of the articles and methods of this invention in photographic systems such as, for example, in photographic films is similar in some respects to use a photoresist. Thus, thin film or collections of films may be employed as either a direct print positive photographic film, or as a negative film. The variety and intensity of colours which are known to be possessed by these layers upon polymerization lend them to colour photography, while their extreme resolution and sensitivity result in photographic materials of unparalleled resolving power and efficiency.

Briefly stated, the compositions useful in the articles and methods of the invention comprise photoresistive layers comprising one or more members of the class of chemical compounds known as diacetylenes. Diacetylenes may be seen to possess at least two carbon-carbon triple bonds (acetylenic bonds) at least two of which triple bonds are in conjugation one with another, i.e. exist in a 1—3 relationship as is illustrated:

I. $R_1\ C{\equiv}C{-}C{\equiv}C{-}R_2$

As is known to those skilled in the art, an acetylenic bond possesses a generally linear geometry. It follows that diacetylenes possess a generally linear arrangement of six atoms, the four carbon atoms participating in the diacetylenic "backbone" and each of the two atoms bonded to either end of that backbone. In addition, it is apparent that the diacetylenic structure, being rich with electron density, possesses a novel electronic structure. It is this electronic and geometric structure possessed by the genus of diacetylenes which is believed to contribute in major degree to the unique suitability of such compounds for inclusion in photographic or photolithographic articles as taught by this invention.

Diacetylenes which are suitable for use in the practice of this invention conform to the general formula:

I. $R_1{-}C{\equiv}C{-}C{\equiv}C{-}R_2$

where $R_1$ and $R_2$ may be the same or different and may comprise alkyl, aryl, alkaryl, or aralkyl groups having from one to 50 carbon atoms. $R_1$ and $R_2$ may, in addition, have heteroatomic substitutions or unsaturations. Thus, $R_1$ or $R_2$ may include one or more ester, acid, alcohol, phenol, amine, amide, halogen, sulfonyl, sulfoxyl, sulfinyl, silyl, siloxyl, phosphoro, phosphato, keto, aldehyde, or other moieties. In addition, metal modifications of any of the foregoing may be included such as, for example, acid or phenolate salt. In addition $R_1$ or $R_2$ or both may be ester acid, alcohol, phenol, amine, amide, halogen, sulfonyl, sulfoxyl, silyl, siloxyl, phosphoro, phosphato, keto, aldehyde or a metal salt or phenolate. In short, it is contemplated that any diacetylene may be suitable for use in the practice of one or more of the embodiments of the invention with the exception of those diacetylenes wherein $R_1$ or $R_2$ or both are hydrogen. The latter compounds are not suitable due to the fact that they are, in general, explosive.

It is to be understood that the species referred to in this description of the invention may be either straight chain, cyclic, aromatic, or branched. It should also be understood that reference to the compounds as being diacetylenes does not foreclose the presence of additional acetylenic bonds therein. Thus, compounds having 3, 4 or more acetylenic bonds are foreseen as long as at least two or more such bonds are in conjugation one with another. Furthermore, additional sites of unsaturation may be present such as carbon-carbon, carbon-oxygen, carbon-nitrogen, or other double bonds, aromatic or heteroaromatic species. Substitution with halogens, hydroxyls, amines, thiols, silyls, siloxyls, phosphates, sulfates, sulfonates, or other functionalities is also useful.

For the practice of certain embodiments of the invention, diacetylenes may preferably possess the general formula:

II. $R_3{-}C{\equiv}C{-}C{\equiv}C{-}R_4$

wherein $R_3$ is a hydrophobic chemical moiety and $R_4$ is a hydrophilic chemical moiety. Those skilled in the art will recognize that "hydrophobic" is a term descriptive of chemical moieties or residues which are, in general, unattracted to water or electrically charged species. Thus, hydrocarbon structures which are unsubstituted or sparingly substituted with heteroatomic functionalities are considered hydrophobic. In contrast, a "hydrophilic" moiety is one which is attracted to water or electrically charged species and is considered to be one which is substituted with heteroatomic substituents. Thus, hydrophilic species possess one or more acid, ester, alcohol, amino, thiol, or similar heteroatomic substituents while hydrophobic species are characterised by a substantial lack thereof. Of special utility in the practice of the invention are diacetylenes of formula (II) wherein the substituent $R_3$ comprises a hydrocarbon moiety having from one to about 30 and preferably from 2 to about 20 carbon atoms and wherein $R_4$ may be represented by the formula:

III. $-R_5-(A)_n$

wherein $R_5$ is a hydrocarbon having from one to about 50 and preferably from one to about 30 carbon atoms; n is an integer from one to about

10 and preferably one to three; and A is a member of the group consisting of halogen, COOH, $COOR_6$, $CONH_2$, $CONHR_6$, OH, SH, $NH_2$, $NHR_6$, $N(R_6)_2$, silyl, siloxyl, sulfate, sulfonate, phosphate and others where $R_6$ is a hydrocarbon having from one to about 8 carbon atoms. Certain preferred forms of the composition include styryl moieties in the hydrocarbon portion of $R_3$ or include other polymerizable unsaturations such as, for example, dienyl, vinyl or acrylic species.

Certain embodiments of the invention may usefully be accomplished utilizing compounds of the formula:

IV. $R_3{-}C{\equiv}C{-}C{\equiv}C{-}R_3$

or

V. $R_4{-}C{\equiv}C{-}C{\equiv}C{-}R_4$

where R and R have any of the identities attributed to them above.

Additional embodiments of the invention may profitably employ diacetylenes of formula (I) where $R_1$ or $R_2$ or both have the formula:

VI. 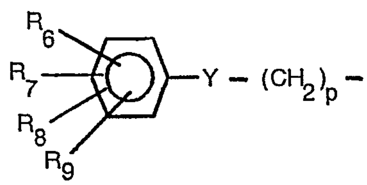

where p is an integer from 0 to about 20 preferably from one to about 10; Y is 0, NH, S, $SO_2$, $SO_3$, $SiO_2$, $PO_3$, $PO_4$, $CH_2$, amido, acetyl, acetoxy, acrylyl, methacrylyl, or styryl; and $R_6$ through $R_9$ may be the same or different and may be H; $NO_2$; $NH_2$; monohalomethyl; dihalomethyl trihalomethyl; halogen, alkyl, alkenyl, or aryl having from one to about 6 carbon atoms; $SO_2$, $SO_3$, $PO_3$, $PO_4$, siloxyl, silyl etc. In certain preferred compounds, ethylenic groups are included to result in styryl diacetylenic formulations. In certain compounds, centers of chirality or asymmetry may be present in the molecular structures and optically active compounds may be utilized for certain embodiments. Thus, compounds may be utilized such as, for example, those of formula (I) wherein $R_1$ or $R_2$ or both have the formula:

VII. $-(CH_2)_q-R_{10}$

where q is an integer from 0 to about 20 and $R_{10}$ is a species having a chiral or optically active center. While it is to be understood that any substituent having an optical centre is contemplated for use herein, several exemplary embodiments may be represented by the formulae:

VIII. 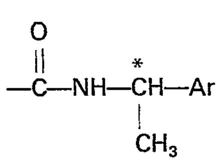

IX. 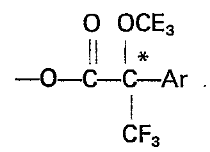

or

X. 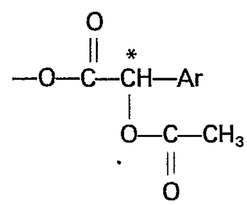

wherein an asterisk indicates an optical centre and Ar represents an aromatic group, preferably phenyl or naphthyl. In similar fashion, chiral amino acid or other optically active residues may be included in the diactylenic compounds of the invention.

In some embodiments of the invention, especially those in which polymerization by UV or X-ray radiatiof is desired, it has been found convenient to include a number of heteroatomic species in the compositions of the invention. Thus halogens, especially fluorine, sulfur, selenium, phosphorus and similar atoms are preferably included. This is done to improve the cross section of energy which may be absorbed by the composition, thus to improve its overall efficiency. Of special note is the employment of polyfluoroalkyl substituents in the practice of the invention for this purpose; specific use of such moieties is contemplated hereby.

It should be apparent from the foregoing that no limitation is intended or is to be implied with respect to the diacetylenes suitable for the practice of one or more embodiments of this invention. All compositions which include one or more chemical compounds having at least two acetylenic bonds, at least two of which are in conjugation one with another are suitable.

Exemplary syntheses of diacetylenes are presented in "Synthesis of N-(nitrophenyl)amine Substituted Diacetylene Monomers". Garito et al, Makromolecular Chemie (in press); "Synthesis of Chiral Diacetylene Polymers", Garito et al, Makromolecular Chemie (in press); "The Chemistry of Diacetylenes" Keter Pub. House; Jerusalem (1974); "Synthetis of Nitrophenoxymethyl Substituted Diacrylene Monomers", Kalyanaraman, Garito et al, Makromolecular Chemie, vol. 180, June 1979; "Solid-State Synthesis and Properties of the Polydiacetylenes", Baughman et al, Annals of NY Academy of Science, vol 313, (1978); "Polymerization of Diacetylene Carbomic Acid Monolayers at the Gas-Water Interface", Day et al, J. Polymer Sciences, Polymer Letters ed. vol. 16, p. 205 (1978); and U.S. Patent 3,923,622 issued to Baughman et ag.

As a class, diacetylenes exhibit uniquely regular structure in thin films, multi-layer films, and polymers formed therefrom. In thin films formed on substrates, diacetylenes assume a regular

orientation. Upon polymerization, this regular orientation is, to a large degree, maintained resulting in polymers having a regular orientation. This phenomenon which is illustrated in Fig. 1 is known.

The chemical molecular structure of such polymers, while not entirely clear, is subject to interpretation. As shown in Fig. 1, polymers of diacetylenes are believed to possess triple and double bonds in a 1—3 relationship in the subunits of the polymer. It will be understood by those skilled in the art that the two "resonance structures" indicated for the polymer represents the fact that, with poly(diacetylenes) as with most organic molecules, structural description in terms of bond order, i.e. triple, double, etc. is less than precise. Thus, with the understanding that the polymers possess bond characteristics which are not fully representable as any one single structure, such polymers will be described as having an acetylenic bond in the subunit thereof. The polymers may be alternatively described as having a repeating subunit wherein four carbon atoms are aligned in a generally linear configuration. Thus, the polymers produced according to the practice of this invention may be alternatively described as 1) being substantially regular in orientation, at least within any polymer domain; 2) having an acetylenic bond in the subunit structure thereof, and 3) possessing subunits which have four carbon atoms in a generally linear configuration.

In general, it is possible to coat substrates with diacetylenes and to utilize such coatings for photo-resistive and other purposes. It is believed that the regular orientation of the diacetylenic "backbone" in the layers so formed places the acetylenic bonds in the proper orientation for polymerization to proceed with high efficiency. This factor combined with the amenability of diacetylenes to absorb radiant energy and to undergo photolytic generation of reactive intermediates such as radicals or carbenes causes such oriented arrays of diacetylenes to be highly reactive towards incident radiation and to effect polymerization quickly upon irradiation.

Coating of substrates with diacetylenic compositions is preferably accomplished by the Langmuir-Blodgett technique. This technique, which is well known to those skilled in the art, causes a thin film of diacetylene to be deposited upon the surface of a fluid. The surface layer is then compressed to minimize the surface area occupied by the diacetylene so as to effect a closest packing arrangement thereof. This closely packed and arrayed diacetylenic layer is then transferred to a substrate by dipping. The use of diacetylenes having hydrophobic and hydrophilic substituents on either end thereof facilitates the use of the technique. The layers may number from two to several hundreds and may, thus, comprise thin or thick films.

Alternative means of placing diacetylenes on substrates may be utilized as well. Thus, the "whirling" technique as described in the DeForest reference, roller coating as is currently practiced in the art, or even dipping may be employed so as to apply the diacetylenic species to the substrate. Coating by vapour deposition may also be employed. The thickness of the films employed in the articles of the invention may vary depending upon the use contemplated therefore. From a few to about 40 Langmuir-Blodgett constructed layers or a comparable thickness of film formed by one of the alternative means is most suited to photoresistive purposes while greater or lesser thicknesses lend themselves to other embodiments such as photography.

The composition useful in the practice of the invention may include species in addition to the aforedescribed diacetylenes. Thus, additional polymerizable materials may be added as may catalysts, photo-initiators, pigments, and fillers. Additionally, organic or inorganic materials may be included to alter the electrical properties of the compositions, especially for use in thin film devices. The additional polymerizable materials which may be included may encompass any of the wide variety of polymerizable species known to those skilled in the art. Olefinics such as vinyl, styryl, acrylic, dienyl, etc. are preferred. Of these, dienyl and acrylic species are preferred. Dimers of nitroso compounds may also be included as indicated above.

The composition may, optionally, contain a sensitizer or catalyst to improve the photochemical interaction between the compositions and incident radiation. Such sensitizers are well known in the art and include, for example, acetophenone, acyloin derivatives, benzophenone, dyes such as indigo derivatives and many other species. The sensitizers may be included in amounts up to about 5% by weight of composition and preferably between about 1% and about 3%. One or more layers of diacetylenic composition may be "sandwiched" with layers of sensitizer-containing formulation to give good results.

Other compositions may include in the diacetylenic species polymerizable sites in addition to the diacetylenic bonds themselves. Thus, diacetylenic compounds having acrylic, styryl, vinyl or other polymerizable functionalities may be used to good result. In such a case, the polymerization of such additional polymerizable structures may be accomplished concomitantly with or subsequent to the polymerization of the "backbone" diacetylenes. In cases where multiple single layers of oriented diacetylenes are laid down upon a substrate, it may be seen that polymerization of the "backbone" may occur almost exclusively within one layer. The presence of other polymerizable species creates the possibility of interlayer polymerization or cross linking. The inclusion of styrene residues is especially preferred for this purpose.

For the practice of photography and photolithography it is highly desirable to employ photosensitive materials which combine high resolution with high sensitivity to radiation. Such

goal is attained by the present invention through the use of ordered arrays of diacetylenic films of high sensitivity which has been arranged in a plurality of domains.

It is believed that the substantially regular array evidenced by the diacetylenic films according to the practice of this invention, especially those elaborated by the Langmuir-Blodgett technique, contribute to high photoreactivity of those films as reflected in the high quantum efficiency alluded to above. While such high efficiency is to be desired from the point of view of speed of reaction and overall savings of energy in use, it will be appreciated that such high efficiency appears to be contrary to the twin goal of high resolution. An article having the high efficiency of the present invention would be expected to evidence uncontrolled polymerization or reaction upon exposure to radiation and to react not only in exposed areas, but also in neighbouring, unexposed areas as well.

The present invention overcomes this difficulty and provides articles having both a very high efficiency and a high resolving power.

This goal may be obtained through a technique whereby substantially ordered or arrayed films on layers of diacetylenes are caused to be arranged in a plurality of domains within the film or layer structure. It will be appreciated that a substantially regular array of molecules will be retained within the domains thus provided, but that the domains will be randomly oriented so that a domain's molecular array orientation will not normally be repeated in the array orientation of its neighbours. Thus, within any domain, a substantially regular array of diacetylenic molecules will prevail, but that array will terminate at the domain boundary. It will be apparent from the foregoing that the sensitivity of the diacetylenic species arrayed, within a domain will remain largely unchanged, but that a photolytic reaction initiated in one domain will not normally pass over into neighbouring domains. It will be similarly apparent that the above embodiment will yield photographic materials and films of high sensitivity, and resolution.

The arrangement of diacetylenic films into a plurality of domains may be accomplished in a number of ways. Thus a substantially ordered film or layer of diacetylenic material may be heated either conventionally, through laser or microwave heating techniques, or in other ways to accomplish the establishment of the domains. With conventional heating, heating to just below the melting point of the layer is preferred. Alternatively, during the employment of Langmuir-Blodgett film-forming techniques, one may adjust the pH, the temperature, or the specific gravity of the water subphase utilized in the process to cause the assumption of domains.

It is believed that the size of the domains produced in the practice of the invention will be reflected in the ultimate resolving power of the photoresist. In practice it has been possible to create domains of average dimension well below 100 nm but larger and smaller domain sizes are specifically envisioned as well. Thus, preferred embodiment include domains having average dimensions less than about 1,000 nm, and even more preferably less than about 200 nm.

An alternative procedure to the establishment of domains in the diacetylenic layers is to include molecules of lower polymerizability in the compositions. These molecules may be any of a wide range of materials including certain of the less reactive diacetylenes, long chain olefins or fatty acids. Alternatively, diacetylenic species may be chosen which possess steric or electronic structures which are not entirely favourable to polymerization either by upsetting the substantially ordered array present in the film or layer or by presenting molecules of lesser reactivity to the advancing polymer chain. Such inclusions may be seen to "temper" the reactivity of the films or layers and to provide an alternative means of control of the system.

For certain applications, it is highly beneficial to provide a strong adherence of the coatings to the underlying substrate. It is possible to bond such coatings to substrates covalently utilizing certain techniques. Thus, hydroxyl or other functional groups commonly found on the surfaces of most substrates may be utilized to consummate silyl or siloxyl linkages with a suitably silicon substituted diacetylenic species. See E. P. Plueddemann, "Mechanism of Adhesion Through Silane Coupling Agents" in Composite Materials, Brautman, Krock eds, vol. 6, ch. 6, Academy Press (1974). Other means of covalent bonding of film to substrate or of film precursor species to substrate will readily occur to those skilled in the art. Thus, it is desirable to coat the substrate with a composition which may form covalent linkages with such substrate and which may also form covalent linkages with the diacetylenic species which comprise the photoresistive layer. While any compound which will form the covalent bonding may be employed, preferred species for accomplishing such covalent bonding may be represented by the formula:

XI. $(R_{11}—O)_3—Si(R_{12})—Z$

where the $R_{11}$ groups may be the same or different and are hydrocarbyl groups having from 1 to about 6 carbon atoms, where $R_{12}$ is a hydrocarbyl group having from 1 to about 6 carbon atoms, and Z is any substituent which may be covalently bonded with the compound having at least two acetylenic bonds in conjugation with one another. Preferably, Z is an amine, and is used to form an amide linkage with a carboxyl group on the diacetylene, but any suitable substituent may be employed. One such exemplary compound is 3-aminopropyltriethoxysilane which is described by formula (XI) when $R_{11}$ is ethyl, $R_{12}$ is propyl and Z is amino. It will be understood that covalent bonds other than the siloxyl and amide bonds described above may be satisfactorily employed in the practice of the invention.

It has also been found that certain nitroso dimers such as 1-nitrosooctadecane dimer, 1-nitrosododecane dimer and 1-nitroso-cyclohexane dimer may be included in the compositions to retard thermally-induced polymerization without interfering with the photochemical polymerizations central to use of the compositions as photoresists. In this regard, it is believed that the dimer dissociates thermally to species which inhibit polymerization. Thus thermal polymerization is hindered while photochemical polymerization is unaffected. Any other compounds which reacts thermally to provide radical trapping species is also useful in this regard.

It should be apparent that any form of irradiation may be utilized to effectuate the polymerization of the article of the invention. Of the forms commonly used, particle beam, electron beam, X-ray, laser and ultraviolet irradiation are more preferred. As should similarly be apparent, irradiation with masking, with a controlled beam or without effective spatial control, that is, whole surface irradiation may be utilized. In general it is expected that substantial polymerization will occur within irradiated areas. Substantial polymerization occurs when at least 50% of the monomer species become incorporated in polymer moieties. Preferably 75% and even more preferably 85% or greater incorporation is desired. As has been shown, a selective exposure is desirable for photo lithography and microfabrication. Thus either mode of irradiation may be seen to be useful.

For employment as photographic media, it follows that for many uses, response to visible (or infrared) light is frequently desired. Thus the films or other articles may if necessary employ any of the well-known sensitizers to alter the spectral response of the articles. Of course, the diacetylenic species which possess inherent sensitivity in the visible (infrared) region are suitable for such use without the use of a sensitizer.

Example 1

4-Nitrophenyl propargyl ether:

To a solution of propargyl alcohol (10 g) in Dimethyl-sulfoxide (DMSO) (10 ml) $K_2CO_3$ (1.5 g) was added and the mixture kept stirring. 4-Nitrolfluorobenzene (28 g) was added gradually and the mixture stirred at room temperature for three days. It was poured into excess water slightly acidified with HCl. Thin layer chromatography (TLC) showed two spots, one of which was identical with 4-nitrofluorobenzene Fractional crystallization from cyclohexane afforded a pale yellow solid (15.8 g, 50%) m.p. 103—110°C; I.R. (KBr): 3174 (=CH), 1582, 1315, 847 (Ar—$NO_2$) $cm^{-1}$.

Example 2

2,4-Hexadiyn-1,6-diol-bis(4-nitrophenyl) ether

A solution of the material from Example 1 (1 g) in DMSO (15 ml) was added at 55—60°C, with stirring, to a mixture of cuprous chloride (1 g.) and ammonium chloride (3 g) in water (10 ml) with oxygen passing through the solution. After four hours, the mixture was poured into dilute aqueous ammonium hydroxide solution (500 ml). The yellow ppt. was filtered off, washed with cold water, dried and recrystallized from dioxane-ethanol (1.3 g, 70%). m.p. 213°C dec.; I.R. (KBr): 1592, 1336, 847 (Ar—$NO_2$) $cm^{-1}$.

Example 3

2,4 - Hexadiyn - 1,6 - diol - bis - (2,4 - dinitro-5 - fluorophenyl) ether

To a solution of 2,4-hexadiyne-1,6-diol (5 g) in DMSO (15 ml), triethylamine (2 ml) was added. To the stirring solution at room temperature, 2,4-dinitro-1,5-difluoro-benzene (20 g) was added gradually. After stirring overnight at room temperature, the mixture was poured into water, the product filtered off, washed with water, dried and recrystallized from dioxane-water (16 g., 75%). The compound decomposes above 190°C without melting. I.R. (KBr): 1587, 1333, 846 (Ar—$NO_2$), 1176 (Ar—F)$cm^{-1}$.

Example 4

2,4 - Hexadiyn - 1,6 - diol - bis - (2 - nitro - 4-trifluoromethylphenyl) ether

Reaction of 2,4-hexadiyne-1,6-diol (1 g) with 4-fluoro-3-nitro-benzotrifluoride (4 g.) following the procedure of Example 3 yielded the desired product. Recrystallization from ethanol gave pale yellow needles. (1.1 g., 25%) m.p. 137°C. I.R. (KBr): 1620($NO_2$), 1120 682 ($CF_3$)$cm^{-1}$.

Example 5

4-Nitrophenyl 1-Bromopropargyl ether

To a solution of KOBr in water (from 22 g. KOH, 5 ml, $Br_2$ and 100 ml water) at 0°C, 4-nitrophenyl propargyl ether (3.5 g) in dioxane (80 ml) was added dropwise with stirring. After stirring an additional 15 minutes, the solution was poured into excess ice water and the precipitate was filtered off and recrystallized from dioxane as a white powder (4.5 g., 90%), m.p. 156—157°C. I.R. (KBr): 1587, 1324, 847 (Ar—$NO_2$)$cm^{-1}$.

Example 6

1-(4-Nitrophenoxy)2,4-hexadiyne-6-ol

To a suspension of cuprous chloride (20 mg), ethylamine (70%, 1.4 ml) and hydroxyamine hydrochloride (0.12 g) in water (5 ml), a solution of propargyl alcohol (1 g) in DMSO (2 ml) was added with stirring. To the yellow mixture, the bromo ether of Example 5 (0.6 g.) in DMSO (20 ml) was added dropwise at 20°C over 40 mins. After stirring for an additional one hour, the solution was poured into excess water, the yellow precipitate filtered off and recrystallized from methanol as short needles (0.4 g., 74%), m.p. 172—173°C. I.R. (KBr): 3448(−OH), 1582, 1333, 846 (Ar—$NO_2$)$cm^{-1}$.

Example 7

1 - (2,4 - dinitrophenoxy) - 6 - (4 - nitrophenoxy) 2,4 - hexadiyne

The material of Example 6 (0.23 g.) was dissolved in DMSO (3 ml) and triethylamine (0.5 ml) was added to the stirring mixture at room temperature, 2,4-dinitrofluorobenzene (0.2 g.) was added dropwise. After stirring for four hours, the reaction mixture was poured into water, the precipitate filtered off and recrystallized from dioxane to afford pink crystal (0.35 g., 88%), m.p. 125°C. I.R. (KBr): 1589, 1333, 846(Ar—$NO_2$)$cm^{-1}$.

Example 8

N-(2-Propynyl)-2-methyl-4-nitroaniline

To a suspension of potassium carbonate (20.0 g) in DMSO (7 ml) was addded triethylamine (3 ml) and 2-propyn-1-amine (7.10 g, $1.29\times10^{-1}$ mole). 2-Fluoro-5-nitrotoluene (20.00 g, $1.29\times10^{-1}$ mole) was added with stirring and the reaction mixture was heated at 50°C for three days. The mixture was cooled, poured into excess water, and extracted with methylene chloride. The methylene chloride solution was dried over sodium sulfate, filtered and evaporated. The residue was chromotographed on a silica column, eluting with benzene, and recrystallized from benzene to afford yellow crystals; m.p. 121—123°C. Yield: 15.02 g (61%). I.R. (KBr): 3344 (NH), 3226 (≡CH), 1580 and 1282 $cm^{-1}$ (Ar—$NO_2$).

Example 9

N,N' - Bis(2 - Methyl - 4 - nitrophenyl) - 2,4-hexadiyn - 1,6 - diamine

To a suspension of $Cu(OAc)_2.H_2O$ (1.5 g) in pyridine-methanol (1:1, 15 ml) was added N-(2-propynyl)-2-methyl-4-nitroaniline (1.00 g, $5.26\times10^{-3}$ mole). The reaction mixture was stirred at 50°C for two hours. The mixture was poured into excess water and filtered. The crude solid recrystallized from nitromethane to affored yellow crystals. Yield: 0.70 g (71%). The material did not melt below 200°C but turned red at 150°C. I.R. (KBr): 3390 (NH), 1585 and 1280 $cm^{-1}$ (Ar—$NO_2$).

Example 10

N-(2-Propynyl)-4-nitro-2-trifluoromethylaniline

To a suspension of potassium carbonate (20.0 g) in DMSO (7 ml) was added triethylamine (3 ml) and 2-propyn-1-amine (7.92 g, $1.44\times10^{-1}$ mole). The reaction mixture was cooled in an ice bath while 1-fluoro-4-nitro-2-trifluoro-methylbenzene (30.13 g, $1.44\times10^{-1}$ mole) was added gradually with stirring. The mixture was gradually allowed to warm to room temperature and stirred for 24 hours. The reaction mixture was poured into excess water and extracted with ethyl acetate. The ethyl acetate solution was dried over sodium sulfate, filtered and evaporated. The residue was chromotographed on a silica column, eluting with benzene, and recrystallized from benzene to afford yellow crystals; m.p. 71—72°C. Yield: 24.24 g (69%). I.R. (KBr): 3450 (NH), 3290 (=CH), 1588 (Ar—$NO_2$) and 1300 $cm^{-1}$ ($CF_3$).

Example 11

N,N' - Bis(4 - nitro - 2 - trifluoromethylphenyl)-2,4 - hexadiyn - 1,6 - diamine

To a suspension of $Cu(OAc)_2.H_2O$ (4.5 g) in pyridine-methanol (1:1, 30 ml) was added N-(2-propynyl)-4-nitro-2-trifluoromethylaniline (3.01 g, $1.23\times10^{-1}$ mole). The reaction mixture was stirred at 50°C for two hours. The mixture was poured into excess water and filtered. The crude solid was recrystallized from nitromethane to afford pale pink crystals. Yield 2.54 g (84%). The material does not melt below 200°C but turns dark red at 135°C. I.R. (KBr):3405 (NH), 1580 (Ar—$NO_2$) and 1290 $cm^{-1}$ ($CF_3$).

Example 12

N-(2-Propynyl)-2,4-dinitro-5-fluoroaniline

To a suspension of potassium carbonate (6.0 g) in acetone (25 ml) was added triethylamine (1.5 ml) and 2-propyn-1-amine (2.34 g, $4.25\times10^{-2}$ mole). 1,5-Difluoro-2,4-dinitrobenzene (10.00 g, $4.90\times10^{-2}$ mole) was added and the reaction mixture was stirred at room temperature for 24 hours. The mixture was poured into excess water and filtered. The resulting dark solid was chromatographed on a silica column, eluting with benzene, and recrystallized from benzene to afford yellow crystals; m.p. 113—115°C. Yield: 8.33 g (82%). I.R. (KBr): 3340 (NH), 3280 (=CH), 1615, 1260 (Ar—$NO_2$) and 1210 $cm^{-1}$ (Ar—F).

Example 13

N,N' - Bis(2,4 - Dinitro - 5 - fluorophenyl)-2,4 - hexadiyn - 1,6 - diamine

To a suspension of $Cu(OAc)_2.H_2O$ (1.5 g) in pyridine-methanol (1:1, 10 ml) was added N-(2-propynyl)-2,4-dinitro-5-fluoroaniline (1.00 g, $4.18\times10^{-3}$ mole). The reaction mixture was stirred at room temperature for 17 hours. The mixture was poured into excess water and filtered. The crude solid and chromatographed on a silica column, eluting with chloroform ethyl acetate. Soxhlet recrystallization of the resulting solid from ethyl acetate afforded yellow crystals m.p. 225°C dec. Yield: 0.21 g (12%). I.R. (KBr): 3360 (NH), 1680 and 1315 $cm^{-1}$ (Ar—$NO_2$).

Example 14

Preparation of N - d(+)(α - methylbenzyl)-10,12 - pentacosadiynamide

To a solution of 510 mg (1.36 mmol) of 10 12-Pentacosadiynoic acid in 10 ml tetrahydrofurane was added 138 mg (1.36 mmol) of triethylamine. The resulting cloudy solution was cooled to 0°C and 129 mg (1.36 mmol) of methyl chloroformate was added dropwise over 1 min. A white solid formed immediately upon addition. The mixture was stirred 1 hour at 0°C, then 165 mg (1.36 mmol) of d(+)-α-methylbenzylamine was added and the mixture was heated at reflux for one hour. Gas evolution was evident within five minutes of addition, and ceased after 15 minutes. The mixture was cooled to room temperature and filtered. The filtrate was washed with 10 ml portion of 1 molar HCl, water, and

saturated aqueous potassium bicarbonate solution, and dried ($MgSO_4$). Evaporation yielded 540 mg (83% crude yield) of a white solid. Recrystallization from ether-petroleum ether gave white crystals, m.p. 65.5—66.5°C. I.R. film; 1470, 1545, 1640, 2860, 2925, and 3310 $cm^{-1}$. $[\alpha]_D^{31}(CHCl_3)=44°$.

Example 15
Preparation of N,N' - bis - (α - methylbenzyl)-10,12 - docosadiyndiamide

To a solution of 725 mg (2.00 mmol) of 10,12-docosadindioic acid in 50 ml Tetrahydrofurane (THF) was added 405 mg (4.00 mmol) of triethylamine. The solution was cooled to 0°C and 378 mg (4.00 mmol) of methylchloroformate was added dropwise over one minute. The resulting white mixture was stirred one hour at 0°C, the 485 mg (4.00 mmol) of d(+,−α-methylbenzylamine was added. The reaction mixture was heated at reflux for one hour (gas evolution began immediately upon amine addition and ceased after 20 minutes). The mixture was cooled to room temperature and filtered. The filtrate was washed with 25 ml portions of 1 molar HCl, water, and saturated aqueous sodium bicarbonate solution and dried ($MgSO_4$). Evaporation yielded 842 mg (74% crude yield) of a white solid, which polymerized rapidly upon exposure to UV light. Recrystallization from ether-petroleum ether gave 456 mg of white crystals, m.p. 87.5—89°C. I.R. (film): 1530, 1635, 2850, 2920, and 3300 $cm^{-1}$.

Example 16
2,4 - Hexadiyn - 1,6 - diol - bis - (2,4 - dinitrophenyl) ether

To a solution of 2,4-hexadiyn-1,6-diol (1.1 g) in acetone (15 ml), $K_2CO_3$ (0.5 g) was added. To the stirred solution at room temperature 2,4-dinitrofluorobenzene (3.8 g) was added gradually and the dark red solution stirred overnight at room temperature. It was poured into excess water, the pale yellow solid filtered off, washed with water and dried. Recrystallization from dioxane ethanol gave short, light pink needles, m.p. 210°C (4.2 g, 95%). I.R. (KBr): 1592, 1333, 834 (Ar—$NO_2$ $cm^{-1}$.

Example 17
N-(2-Propynyl)-2,4-dinitroaniline

To a suspension of potassium carbonate (1.0 g) in acetone (10 ml) was added 2-propyn-1-amine (0.26 g, $4.73\times10^{-3}$ mole). 2,4-Dinitrofluorobenzene (1.32 g, $7.10\times10^{-3}$ mole) was gradually added with stirring and the reaction mixture was refluxed two hours. After cooling it was poured into excess water and filtered. Recrystallization of the crude solid from ethanol afforded yellow needles; m.p. 151—152°C. Yield 0.99 g (95%). I.R. (KBr): 3367 (NH), 3268 (CH), 1618, 1590, 1333 and 1311 $cm^{-1}$ (Ar—$NO_2$).

Example 18
N,N' - Bis(2,4 - Dinitrophenyl) - 2,4 - hexadiyn-1,6 - diamine

To a suspension of $Cu(OAc)_2.H_2O$ (1.5 g) in pyridinemethanol (1:1, 10 ml) was added N - (2 - propynyl) - 2,4 - dinotroaniline (1.00 g, $4.52\times10^{-3}$ mole). The reaction mixture was stirred at 50°C for 30 minutes. The mixture was poured into excess water, and filtered. The crude solid was recrystallized from nitromethane to afford pale green crystals. Yield: 0.86 g (86%). The compound failed to melt below 200°C but changed from green to bronze colour at 120°C. It could be recrystallized from dioxane to give a different crystal form, appearing as orange crystals which turn deep orange at 140°C. I.R. (KBr): 3380 (NH), 1617, 1592, 1333 and 1312 $cm^{-1}$ (Ar—$NO_2$).

Example 19
Synthesis of Diacetylene alkyl-acid monomers

Diacetylene alkyl-acid monomers for use in mono- and multilayer preparations were synthesized by the Chodkiewicz coupling procedure using bromoacetylenes prepared following Strauss. See Chodkiewicz, W. Ann. Chem. (Paris) 2, 853 (1957) and Strauss, et al Ber. 63B, 1868 (1930). For example, 1-bromoundecyn-10-oic acid was coupled to tetradecyne to form pentacosa-10,12-diynoic acid. Fifty mmoles of tetradecyne dissolved in 5 ml ethanol was added with stirring to a 50 ml ethanol solution of 100 mmoles ethylamine forming a yellow solution. The stirred solution was cooled at 15°C and 50 mmoles of 1-bromo-undecyn-10-oic acid dissolved in 60 ml ethanol was added dropwise over 30 minutes while the temperature was maintained at 15—20°C. After the addition was complete, the reaction mixture was stirred for 3 hours at 15—20°C. The mixture was then acidified to pH 1 and extracted twice with 100 ml ethyl acetate. The organic layer was separated, dried over magnesium sulfate, filtered through fluorosil and evaporated to give a colourless, viscous oil. The oil was taken up in methanol-petroleum ether and the solution was filtered. Upon cooling of the filtrate, pentacosa-10,12-diyoic acid crystallized as colourless platelets (m.p. 59—60°C). The platelets become an intense blue upon standing in laboratory light for a short time.

Example 20

The amide produced in Example 14 was elaborated into a fourteen, Y-layer, multilayer assembly by the Langmuir-Blodgett technique on a ferric stearate-coated quartz plate and warmed in an oven at 60°C for two hours followed by slow cooling. The assembly was exposed to either UV or X-ray radiation through a mask giving excellent pattern delineation.

Example 21

In separate experiments, 12-30 layers of pentacosa-10, 12-diynoic acid were deposited on solid substrate plates such as glass, silicon, and evaporated gold films on glass. Spatially defined regions of the multilayers were polymerized by X-ray exposure through a mask to produce negative resist device patterns on the substrate

plates. For example, a 30 multilayer assembly of pentacosa-10,12-dynoic acid on a silicon single crystal substrate plate was positioned behind a metal mask to allow exposure of the multilayer assembly to X-rays through a 5 μm wide by 1 cm long mask line. The total X-ray energy absorbed for polymerization and resultant pattern delineation was approximately 10 millijoules/cm$^3$. After exposure, the remining monomer in the unexposed masked regions were dissolved away by rinsing in an organic solvent such as acetone leaving a well-defined line pattern composed of the pentacose-10,12-diynoic polymer on the silicon substrate. After substrate etching (processing) the polymer was removed by washing in a dilute aqueous HF solution.

Example 22

Preparation of a convalently bound diacetylene on a silicon surface

Silicon plates with an oxide layer .65 μm thick were immersed in concentrated nitic acid for 2 hours. After rinsing with water, the water contact angle ($\alpha_w$) was determined to be 44°. After thorough drying, the plates were treated with vapours of 3-aminopropyltriethoxysilane. The substrate was placed over a boiling solution of 2 ml of the silane in 100 ml dry p-xylene under nitrogen for 16 hours. The substrate was bathed in the vapour, the vapour condensing 5 cm above the substrate. The substrate was rinsed in absolute ethanol and water; $\alpha_w$ was determined to be 45°. The silanated substrate was immersed in a solution of 22 mg (0.06 mmol) of 10,12-pentacosadiynoic acid in 10 ml anhydrous pyridine. A solution of 14 mg (0.07 mmol) of N,N-dicyclohexylcarbodiimide in 1 ml pyridine was added. The wafers were treated for 22 hours at room temperature under nitrogen. The substrate was rinsed with pyridine, ethanol, boiling pyridine and boiling ethanol and dried. The $\alpha_w$ was determined to be 78°.

Example 23

A multilayer assembly of 15 layers of the composition of Example 19 elaborated upon a quartz plate by the Langmuir-Blodgett technique was placed in an over at 55°C for 6 hours and then cooled to room temperature. Pluralities of domains were thus established in the assembly which was found to yield excellent definition and response upon selective exposure to UV or X-ray radiation.

Example 24

Ordinary transparent, flexible film was coated with 15 Langmuir-Blodgett layers of the amide of Example 14. The coated film was heated at 55—60°C for 2 hours followed by cooling. The film was then exposed to a test pattern through a lens to afford excellent definition and resolution having a brilliant purple-bronze colouration.

**Claims**

1. A photographic or photolithographic article comprising a substrate, and at least one monomolecular layer carried by said substrate of a photopolymerizable composition, said layer comprising a material having at least two conjugated acetylenic bonds, characterised in that said layer is formed of a plurality of contiguous discrete domains.

2. An article as claimed in Claim 1 wherein within each of said domains a majority of the molecules of said material are orientated in a regular array.

3. An article as claimed in claim 2 wherein the orientation of the molecular array in one of said domains is transverse to the orientation of the molecular arrays of the majority of the domains contiguous thereto.

4. An article as claimed in any one of the preceding claims wherein the domains have an average dimension of not more than 1,000 nm.

5. An article as claimed in any preceding claim wherein the domains have an average dimension of not more than 200 nm.

6. An article as claimed in any preceding claim wherein the domains have an average dimension of less than 100 nm.

7. An article as claimed in any one of the preceding claims wherein said domains include molecules of lower polymerizability than said material.

8. An article as claimed in any preceding claim wherein said material includes molecules which have a steric structure or groups which reduce the polymerizability thereof.

9. An article as claimed in any preceding claim wherein the said material includes groups which bond covalently with said substrate.

10. An article as claimed in claim 9 wherein the groups are silyl or siloxyl groups.

11. An article as claimed in claim 9 or claim 10 wherein a bonding layer is interposed between said layer of a photopolymerizable composition and said substrate, said bonding layer being convalently bonded at least to said layer of a photopolymerizable composition.

12. An article as claimed in claim 11 wherein the bonding layer comprises a compound having the general formula

$$\text{XI.} \qquad (R_{11}—O)_3—SI(R_{12})—Z$$

where the $R_{11}$ groups may be the same or different and are hydrocarbyl groups having from 1 to 6 carbon atoms, where $R_{12}$ is a hydrocarbyl group having from 1 to 6 carbon atoms, and Z is any substituent which may be covalently bonded with the material having at least two conjugated acetylenic bonds.

13. An article as claimed in claim 12 wherein the compound is 3-aminopropyltriethoxysilane.

14. An article as claimed in any preceding claim wherein said layer of a photopolymerizable composition includes a nitroso dimer.

15. An article according to any preceeding Claim wherein the photopolymerizable compound is characterised by having the general formula:

$$R_1—C{\equiv}C—C{\equiv}C—R_2—(A)_n$$

wherein $R_1$ is a hydrophobic moiety having from 1 to 30 carbon atoms and $R_2$ is a substituted hydrophilic hydrocarbyl group having from 1 to 30 carbon atoms, n is an integer from 1 to 6 and A is selected from one or more of —COOH, halogen —$COOR_4$, —$CONH_2$, —$CONHR_4$, —$CON(R_4)_2$, —SH, —$NH_2$, —$NHR_4$, —$N(R_4)_2$, silyl, sulphate, sulphonate, phosphate and siloxyl, in which $R_4$ is a hydrocarbyl group having from 1 to 10 carbon atoms.

16. A method for the production of a photographic or photolithographic article comprising laying down a suitable substrate, forming at least one monomolecular layer of a photo polymerizable composition thereon, said composition comprising a material having at least two conjugated acetylenic bonds, characterized in that said article is heated to a temperature just below the melting point of said material to establish the discrete domains in the layer after coating.

17. A method for the production of a photographic or photolithographic article comprising laying down a suitable substrate, forming at least one monomolecular layer of a photopolymerizable composition thereon, said composition comprising a material having at least two conjugated acetylenic bonds, characterized in that the forming of a molecular layer is carried out using the Langmuir-Blodgett film forming technique, and in that the pH, the temperature and/or the specific gravity of the water sub-phase thereof is adjusted to cause or allow formation of domains in the resulting layer.

18. A method as claimed in either of claims 16 or 17 wherein the process conditions are chosen so that having an average dimension in the plane of the layer of not more than 1,000 nm are formed.

19. A method as claimed in any one of Claims 16 to 18 wherein the substrate is coated with a bonding layer prior to coating with said photopolymerizable composition said bonding layer being capable of forming covalent bonds with said photopolymerizable layer.

20. A method as claimed in Claim 19 wherein said bonding layer includes a compound having the general formula

XI. $(R_{11}—O)_3—SI(R_{12}—Z$

where the $R_{11}$ groups may be the same or different and are hydrocarbyl groups having from 1 to 6 carbon atoms, where $R_{12}$ is a hydrocarbyl group having from 1 to 6 carbon atoms, and Z is any substituent which may be covalently bonded with the material having at least two conjugated acetylenic bonds.

**Patentansprüche**

1. Photographischer oder photolithographischer Gegenstand, umfassend ein Substrat und mindestens eine von dem Substrat getragene, monomolekulare Schicht einer photopolymerisierbaren Zusammensetzung, wobei die Schicht ein Material mit mindestens zwei konjugierten, acetylenischen Bindungen umfaßt, dadurch gekennzeichnet, daß die Schicht aus einer Vielzahl benachbarter, diskreter Bereiche gebildet ist.

2. Gegenstand nach Anspruch 1, wobei innerhalb jedem der Bereiche die Mehrzahl der Moleküle des Materials in einer regelmäßigen Anordnung orientier sind.

3. Gegenstand nach Anspruch 2, wobei die Orientierung der molekularen Anordnung in einem der Bereiche zur Orientierung der molekularen Anordnungen der Mehrzahl der dazu benachbarten Bereiche querverlaufend ist.

4. Gegenstand nach mindestens einem der vorangehenden Ansprüche, wobei die Bereiche eine Durchschnittsgröße von nicht mehr als 1 000 nm besitzen.

5. Gegenstand nach mindestens einem der vorangehenden Ansprüche, wobei die Bereiche eine Durchschnittsgröße von nicht mehr als 200 nm besitzen.

6. Gegenstand nach mindestens einem der vorangehenden Ansprüche, wobei die Bereiche eine Durchschnittsgröße von weniger als 100 nm besitzen.

7. Gegenstand nach mindestens einem der vorangehenden Ansprüche, wobei die Bereiche Moleküle mit geringerer Polymerisationsfähigkeit als das Material beinhalten.

8. Gegenstand nach mindestens einem der vorangehenden Ansprüche, wobei das Material Moleküle mit räumlicher Struktur oder sterischen Gruppen, die dessen Polymerisationsfähigkeit verringern, beinhaltet.

9. Gegenstand nach mindestens einem der vorangehenden Ansprüche, wobei das Material Gruppen beinhaltet, die sich kovalent mit dem Substrat verbinden.

10. Gegenstand nach Anspruch 9, wobei die Gruppen Silyloder Siloxylgruppen sind.

11. Gegenstand nach Anspruch 9 oder 10, wobei zwischen der Schicht aus einer photopolymerisierbaren Zusammensetzung und dem Substrat eine Bindeschicht angeordnet ist, wobei diese Bindeschicht mindestens an die Schicht aus einer photopolymerisierbaren Zusammensetzung kovalent gebunden ist.

12. Gegenstand nach Anspruch 11, wobei die Bindeschicht eine Verbindung der allgemeinen Formel

XI. $(R_{11}—O)_3—SI(R_{12})—Z$

umfaßt, worin bedeuten:

$R_{11}$ gleiche oder voneinander verschieden Kohlenwaserstoffgruppen mit 1 bis 6 Kohlenstoffatomen,

$R_{12}$ eine Kohlenwasserstoffgruppe mit 1 bis 6 Kohlenstoffatomen und

Z irgendein Substituent, der kovalent an das

mindestens zwei konjugierte, acetylenische Bindungen besitzende Material gebunden sein kann.

13. Gegenstand Anspruch 12, wobei die Verbindung 3-Aminopropyltriethoxysilan ist.

14. Gegenstand nach mindestens einem der vorangehenden Ansprüche, wobei die Schicht aus einer photopolymerisierbaren Zusammensetzung ein Nitroso-Dimer beinhaltet.

15. Gegenstand nach mindestens einem der vorangehenden Ansprüche, wobei die photopolymerisierbare Verbindung durch die allgemeine Formel

$$R_1—C{\equiv}C—C{\equiv}C—R_2—(A)_n$$

gekennzeichnet ist, worin bedeuten:

$R_1$ ein hydrophober Rest mit 1 bis 30 Kohlenstoffatomen,

$R_2$ eine substituierte, hydrophile Kohlenwasserstoffgruppe mit 1 bis 30 Kohlenstoffatomen,

n eine ganze Zahl von 1 bis 6 und

A wird gewählt aus einem oder mehreren aus $—COOH$, Halogen, $—COOR_4$, $—CONH_2$, $—CONHR_4$, $—CON(R_4)_2$, $—SH$, $—NH_2$, $—NHR_4$, $—N(R_4)_2$, Silyl, Sulphat, Sulphonat, Phosphat und Siloxyl, worin

$R_4$ eine Kohlenwasserstoffgruppe mit 1 bis 10 Kohlenstoffatomen darstellt.

16. Verfahren zur Herstellung eines photographischen oder photolithographischen Gegestands, umfassend das Auslegen eines geeigneten Substrats, Ausbilden mindestens eine monomolekularen Schicht aus einer photopolymerisierbaren Zusammensetzung hierauf, wobei die Zusammensetzung ein Material mit mindestens zwei konjugierten, acetylenischen Bindungen umfaßt, dadurch gekennzeichnet, daß der Gegenstand auf eine Temperatur gerade unterhalb des Schmelzpunktes des Materials erhitzt wird, um diskrete Bereiche in der Schicht nach dem Beschichten auszubilden.

17. Verfahren zur Herstellung eines photographischen oder photolithographischen Gegenstands, umfassend das Auslegen eines geeigneten Substrats, Ausbilden mindestens einer monomolekularen Schicht aus einer photopolymerisierbaren Zusammensetzung darauf, wobei die Zusammensetzung ein Material mit mindestens zwei konjugierten acetylenischen Bindungen umfaßt, dadurch gekennzeichnet, daß das Ausbilden einer molekularen Schicht unter Anwendung der Langmuir-Blodgett-Filmherstellungstechnik durchgeführt wird und daß der pH, die Temperatur und/oder das spezifische Gewicht deren Wasser-Unterphase so eingestellt werden, um die Ausbildung der Bereiche in der resultierenden Schicht zu verursachen oder zu ermöglichen.

18. Verfahren nach Anspruch 16 und/oder 17, wobei die Verfahrensbedingungen so gewählt werden, daß Bereiche mit einer Durchschnittsgröße in der Schichtebene von nicht mehr als 1 000 nm gebilder werden.

19. Verfahren nach mindestens einem der Ansprüche 16 bis 18, wobei das Substrat vor dem Beschichten mit der photopolymerisierbaren Zusammensetzung mit einer Bindeschicht beschichtet wird, wobei die Bindeschicht in der Lage ist, mit der photopolymerisierbaren Schicht kovalente Bindungen auszubilden.

20. Verfahren nach Anspruch 19, wobei die Bindeschicht eine Verbindung der allgemeinen Formel

$$\text{XI.} \qquad (R_{11}—O)_3—Si(R_{12})—Z$$

beinhaltet, worin bedeuten:

$R_{11}$ gleiche oder voneinander verschiedene Kohlenwasserstoffgruppen mit 1 bis 6 Kohlenstoffatomen,

$R_{12}$ eine Kohlenwasserstoffgruppe mit 1 bis 6 Kohlenstoffatomen und

Z iregendein Substituent, der mit dem mindestens zwei konjugierte, acetylenische Bindungen besitzenden Material kovalent gebunden sein kann.

**Revendications**

1. Article photographique ou photolithographique comprenant un substrat et au moins une couche monomoléculaire portée par le substrat d'une composition photopolymérisable, la couche comprenant une matière ayant au moins deux liaisons acétyléniques conjugées, caractérisé en ce que cette couche est formée de plusieurs domaines discrets contigus.

2. Article selon la revendication 1 où dans chacun de ces domains une majorité des molécules de la matière est orientée en une série régulière.

3. Article selon la revendication 2 où l'orientation de la série moléculaire dans un des domaines est transversale par rapport à la l'orientation des séries moléculaires de la majorité des domaines qui y sont contigus.

4. Article selon l'une quelconque des revendications précédentes où les domaines ont une dimension moyenne ne dépassant pas 1000 nm.

5. Article selon l'une quelconque des revendications précédentes où les domaines ont une dimension moyenne ne dépassant pas 200 nm.

6. Articles selon l'une quelconque des revendications précédentes où les domaines ont une dimension moyenne inférieure à 100 nm.

7. Article selon l'une quelconque des revendications précédentes où les domaines comprennent des molécules de plus faible aptitude à la polymérisation que la matière.

8. Article selon l'une quelconque des revendications précédentes où la matière inclut des molécules ayant une structure stérique ou des groupes qui en réduisent la l'aplitutde à la polymérisation.

9. Article selon l'une quelconque des revendications précédentes où la matière comprend des groupes qui se lient de façon covalente au substrat.

10. Article selon la revendication 9 où les groupes sont des groupes silyle ou siloxyle.

11. Article selon les revendications 9 et 10 où l'on interpose une couche di liaison entre la couche d'une composition photopolymérisable et le substrat, la couche de liaison étant liée de façon covalente au moins à la couche d'une composition photopolymérisable.

12. Article selon la revendications 11, où la couche de liaison comprend un composé de formule générale

XI. $(R_{11}—O)_3—SI(R_{12})—Z$

où les groupes R11 peuvent être semblables ou différents et sont des groupes hydrocarbyle ayant de 1 à 6 atomes de carbone, où R12 est un groupe hydrocarbyle ayant de 1 à 6 atomes de carbone, et Z est n'importe quel substituant qui peut être lié de façon covalente à la matière ayant au moins deux liaisons acétyléniques conjuguées.

13. Article selon la revendication 12 où le composé est le 3-aminopropyltriéthoxysilane.

14. Article selon l'une quelconque des revendications précédentes où la couche d'une composition photopolymérisable comprend un dimère nitroso.

15. Article selon l'une quelconque des revendications précédentes où le composé photopolymérisable se caractérise en ce qu'il a la formule générale:

$$R1—C{\equiv}C—C{\equiv}C—R2—(A)_n$$

où R1 est une fraction hydrophobe ayant de 1 à 30 atomes de carbone et R2 est un groupe hydrocarbyl hydrophile substitué ayant de 1 à 30 atomes de carbone, n est un nombre entier allant de 1 à 6 et A est choisi parmit un ou plusieures groupes —COOH, halogène-COOR4, $—CONH_2$, —CONHR4, $—CON(R4)_2$, —SH, $—NH_2$, —NHR4, $—N(R4)_2$, silyle sulfate, sulfonate, phosphate et siloxyle, où R4 est un groupe hydrocarbyl ayant de 1 à 10 atomes de carbone.

16. Procédé pour la production d'un article photographique ou photolithographique dans lequel on dépose un substrat approprié, formant au moins une couche monomoléculaire d'une composition photopolymérisable sur ce substrat, la composition comprenant une matière ayant au moins deux liaisons acétyléniques conjugées, caractérisé en ce qu'on chauffe l'article à une température juste inférieure au point de fusion de ladite matière pour établir des domains discrets dans la couche après revêtement.

17. Procédé pour la production d'un article photographique ou photolithographique dans lequel on dépose un substrat approprié, on forme au moins une couche monomoléculaire d'une composition photopolymérisable sur ce substrat, la composition comprenant une matière ayant au moins deux liaisons acétyléniques conjuguées caractérisé en ce qu'on conduit la formation d'une couche moléculaire en utilisant la technique de formation de pellicule de Langmuir-Blodgett, et en ce qu'on ajuste le pH, la température et/ou la densité de sa sous-phase aqueuse pour provoquer ou permettre la formation de domaines dans la couche résultante.

18. Procédé selon l'une quelconque des revendications 16 ou 17 où les conditions du procédé sont choisies de manière qu'il se forme des domaines ayant une dimension moyenne dans le plan de la couche ne dépassant pas 1000 nm.

19. Procédé selon l'une quelconque des revendications 16 à 18 où le substrat est revêtu avec une couche de liaison avant le revêtement avec ladite composition photopolymérisable, la couche de liaison étant capable de former des liaisons convalentes avec ladite couche photopolymérisable.

20. Procédé selon la revendication 19 où ladite couche de liaison comprend un composé de formule générale

XI. $(R_{11}—O)_3—Si(R_{12})—Z$

où les groupes R12 peuvent être semblables ou différents et sont des groupes hydrocarbyle ayant de 1 à 6 atomes de carbone où R12 est un groupe hydrocarbyle ayant de 1 à 6 atomes de carbone, et Z est tout substituant qui peut être lié de façon covalente à la matière ayant au moins deux liaisons acétyléniques conjuguées.

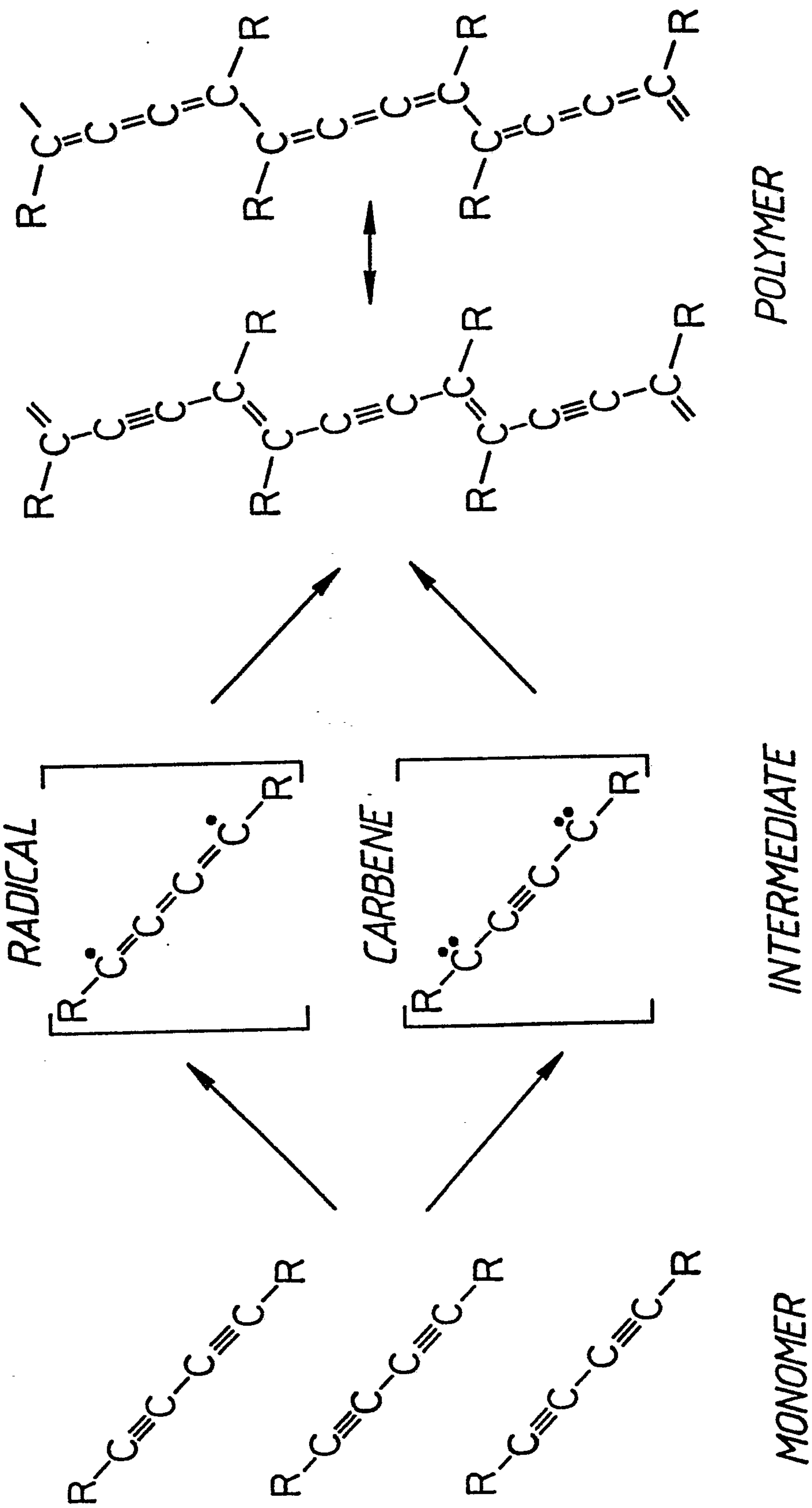


FIG.1

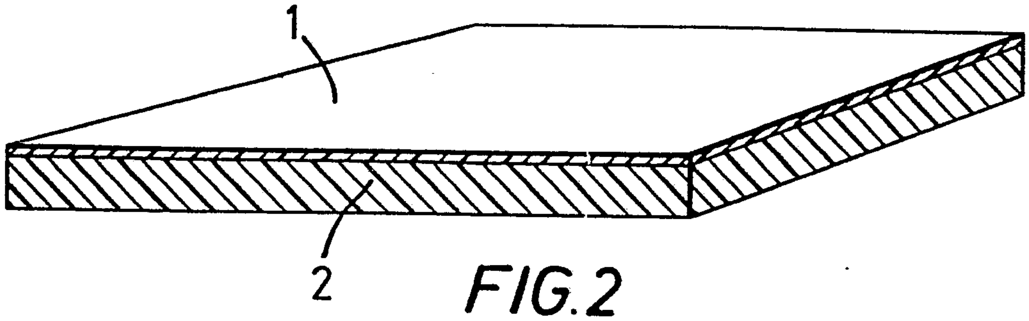


FIG.2

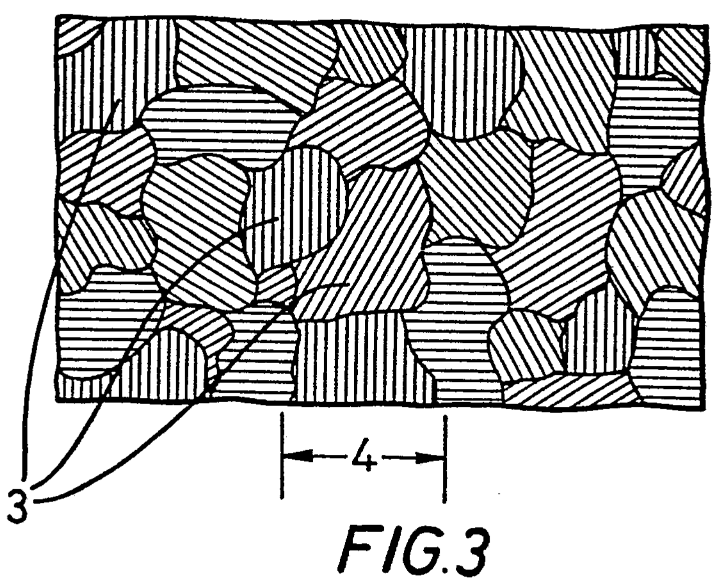


FIG.3